# EUROPÄISCHE PATENTSCHRIFT

(11) **EP 0 599 803 B1**
(45) Veröffentlichungstag und Bekanntmachung des Hinweises auf die Patenterteilung: **05.04.2000**
(21) Anmeldenummer: 93890224.4
(22) Anmeldetag: 15.11.1993
(51) Int. Cl.: G01N 33/543, G01N 33/566, G01N 33/58, G01N 33/553

(54) **Verfahren zum Nachweisen von Antikörpern und Antigenen**
Process for detecting antibodies and antigens
Procédé de décèlement d'anticorps et d'antigènes

(30) Priorität: 23.11.1992 AT 232192; 18.01.1993 AT 6593
(43) Veröffentlichungstag der Anmeldung: 01.06.1994
(73) Patentinhaber: Sanochemia Pharmazeutika AG, 1090 Wien (AT)
(72) Erfinder: Schinkinger, Manfred, Dr., A-2700 Wiener Neustadt (AT); Korger, Markus, Dr., A-1020 Wien (AT)
(74) Vertreter: Beer, Manfred, Dipl.-Ing.

(56) Entgegenhaltungen:
- EP-A- 0 317 001
- WO-A-89/06543
- WO-A-89/06801

## Beschreibung

Die Erfindung betrifft ein Verfahren zum Nachweisen eines in einer Probe enthaltenen Antikörpers durch Binden des nachzuweisenden Antikörpers an ein bezüglich des nachzuweisenden Antikörpers spezifisches Antigen.

Die Erfindung betrifft weiters ein Verfahren zum Nachweisen eines in einer Probe enthaltenen Antigens durch Binden des nachzuweisenden Antigens an ein Fab- oder F(ab)₂-Fragment eines für das nachzuweisende Antigen spezifischen, monoklonalen Antikörpers.

In der WO 89/06801 wird ein Verfahren für die qualitative oder quantitative Bestimmung eines Analyten in einer Probe beschrieben, bei dem ein auf einer festen Phase immobilisierter Reaktionspartner verwendet wird. Dabei soll der Reaktionspartner als Indikator Gold enthalten, das in Teilchenform mit einem mittleren Durchmesser von weniger als 5 nm in einem stöchiometrischen Verhältnis von 1:1 zum Reaktionspartner vorliegen soll.

Die in der WO 89/06801 beschriebene Arbeitstechnik sieht vor, daß der Träger mit der Probe vereinigt und dann inkubiert wird und erst dann eine den Indikator enthaltende Substanz zugegeben wird. Dabei wird auch vorgeschlagen, als Indikator einen Komplex aus Goldpartikeln und Proteinen einzusetzen.

Das erfindungsgemäße Nachweisverfahren von Antikörpern ist dadurch gekennzeichnet, daß man die Probe in ein Reaktionsgemisch, das einen partikelförmigen Träger, an den das bezüglich des nachzuweisenden Antikörpers spezifische Antigen immobilisiert ist, und ein Konjugat aus wenigstens einem zum Fc-Fragment von Antikörpern reaktiven Protein und einen an dieses gebundenen Indikator enthält, einträgt und inkubiert, um Komplexe aus Konjugat und Antikörpern zu bilden und diese Komplexe an den Träger zu binden, daß man hierauf den Träger abtrennt und daß man auf das Vorhandensein von an den Träger gebundenen Komplexen aus Konjugat und dem nachzuweisenden Antikörper prüft.

Bei dem erfindungsgemäßen Verfahren wird die Probe in ein Reaktionsgemisch, das bereits das an einen partikelförmigen Träger immobilisierte Antigen und das Konjugat aus wenigstens einem bezüglich des Antikörpers, d.h. dessen Fc-Fragment reaktiven Protein und einem an diesen gebundenen Indikator enthält, eingetragen, wobei in einer Stufe durch Inkubieren die Aggregate aus dem gebildeten Komplex (aus in der Probe enthaltenen Antikörpern und Konjugat bestehend aus Protein und Indikator, dabei kann ein stöchiometrisches Verhältnis größer 1:2, vorzugsweise 1:20 bis 1:70 vorliegen) und dem am partikelförmigen Träger immobilisierten Antigen gebildet werden.

Die Arbeitstechnik gemäß der Erfindung hat den Vorteil, daß bei der Erfindung auch nicht nachzuweisende Antikörper an der Bildung des Komplexes aus Konjugat und Antikörpern, die in der Probe enthalten sind, teilnehmen, so daß diese Komplexe größer sind, als wenn sie nur aus nachzuweisenden Antikörpern bestünden. Dies insbesondere, wenn das oben erwähnte stöchiometrische Verhältnis eingehalten wird. Der beschriebene erfindungsgemäße Verstärkungsmechanismus ergibt eine interne Signalverstärkung, so daß der Nachweis einfacher und zuverlässiger auszuwerten ist. Dieser Verstärkungseffekt tritt beim erfindungsgemäßen Verfahren auch deswegen auf, weil sowohl die Reaktion zwischen dem Konjugat und dem Antikörper oder den Antikörpern als auch das Binden der dabei gebildeten, den nachzuweisenden Antikörper enthaltende Komplexe gleichzeitig und in einem einzigen Reaktionsmilieu vor sich geht.

Das erfindungsgemäße Verfahren ist aus diesem und, weil es keine zusätzlichen immunchemischen Reaktionen zur Detektion erfordert, Grund besonders einfach ausführbar.

Daher ist bei der Erfindung ein sehr einfacher Nachweisschritt möglich, nämlich ein einfaches Abtrennen (Filtrieren) des Reaktionsgemisches, wobei die Aggregate auf der für die Filtration verwendeten Membran zurückgehalten werden. In diesem Zusammenhang kann darauf hingewiesen werden, daß bei der WO 89/06801 auch Träger bzw. kleinere Aggregate durch die Membran gehen, so daß der Nachweis erschwert ist. Da hingegen bei der Erfindung auch Antikörper, die in der Probe enthalten, aber nicht nachzuweisen sind, die Größe der Komplexe und damit die der Aggregate aus Komplex und Träger erhöhen, wird nicht nur das Abtrennen leichter, sondern es kann auch mit größerer Sicherheit festgestellt werden, ob eine positive Reaktion (= Bindung von den nachzuweisenden Antikörper enthaltenden Komplexen am Träger) aufgetreten ist.

Ein weiterer Vorteil des erfindungsgemäßen Nachweisverfahrens besteht darin, daß dieses in sehr kurzer Zeit und in einem einzigen Verfahrensschritt ausführbar ist, da eine simultane Reaktion aus Konjugat und Probe zur Bildung des Komplexes und aus Komplexen und Trägern mit immobilisiertem Antigen zur Bildung der Aggregate möglich ist. Hingegen wird bei dem Verfahren der WO 89/06801 in mehreren Stufen gearbeitet.

Zum Nachweis von Antikörpern verwendbare Reaktionsgemische sind gemäß der Erfindung dadurch gekennzeichnet, daß das Reaktionsgemisch in einem flüssigen Medium, insbesondere einer Pufferlösung einen partikelförmigen Träger, an dem ein bezüglich des nachzuweisenden Antikörpers spezifisches Antigen immobilisiert ist, und ein Konjugat aus wenigstens einem zum Fc-Fragment von Antikörpern reaktiven Protein und einem an dieses gebundenen Indikator enthält.

Diese Reaktionsgemische können gemäß der Erfindung dadurch hergestellt werden, daß man Trägerpartikel, insbesondere Latexpartikel, vorzugsweise solche aus Polystyrol, mit einem durchschnittlichen Durchmesser von vorzugsweise 1 µm, in einem, vorzugsweise alkalischen, Beschichtungspuffer mit wenigstens einem Antigen beschichtet, daß man die beschichteten Trägerpartikel in einer Pufferlösung, insbesondere einer physiologischen Pufferlösung, in einer Menge von 1% bezogen auf die Pufferlösung suspendiert und daß man der so erhaltenen Suspension wenigstens ein Konjugat aus wenigstens einem bezüglich des Fc-Fragmentes von Antikörpern reaktiven Protein gekoppelt an einen Indikator zumischt.

Das erfindungsgemäße Nachweisverfahren für Antigene ist dadurch gekennzeichnet, daß man die Probe in einem Reaktionsgemisch, das einen partikelförmigen Träger, an dem Fab- oder F(ab)₂-Fragmente eines für das nachzuweisende Antigen spezifischen monoklonalen Antikörpers immobilisiert sind, und ein Konjugat, das aus wenigstens einem zum Fc-Fragment von Antikörpern reaktiven Protein und einem an dieses gebundenen Indikator besteht, sowie wenigstens einen bezüglich des nachzuweisenden Antigens spezifischen monoklonalen Antikörper enthält, inkubiert, um Aggregate aus partikelförmigem Träger, dem nachzuweisenden Antigen und Komplexen bestehend aus Konjugat und Antikörpern zu bilden, und daß man dann auf die Anwesenheit von Aggregaten prüft, die das nachzuweisende Antigen enthalten.

Ein erheblicher Vorteil des erfindungsgemäßen Verfahrens zum Nachweisen von Antigenen liegt darin, daß sowohl die Reaktion zwischen Konjugat, Antikörper und dem Antigen oder den Antigenen als auch das Binden der dabei gebildeten, das nachzuweisende Antigen enthaltenden Komplexe gleichzeitig und in einem einzigen Reaktionsmilieu vor sich geht.

Das erfindungsgemäße Verfahren zum Nachweisen von Antigenen ist u.a. deswegen besonders einfach ausführbar, weil es keine zusätzlichen immunchemischen Reaktionen zur Detektion des Antigens erfordert.

Dem erfindungsgemäßen Verfahren zum Nachweisen von Antikörpern und jenem zum Nachweisen von Antigenen liegt der gemeinsame Gedanke zugrunde, daß Antikörper, die in der Probe enthalten, aber nicht nachzuweisen sind, die Größe der Komplexe und damit die der Aggregate aus Komplex und Träger erhöhen, so daß nach dem Abtrennen leichter und mit größerer Sicherheit festgestellt werden kann, ob eine positive Reaktion (= Binden von das nachzuweisende Antigen bzw. den nachzuweisenden Antikörper enthaltenden Komplexen am Träger) aufgetreten ist.

Zum Nachweis von Antigenen verwendbare Mittel sind gemäß der Erfindung dadurch gekennzeichnet, daß das Mittel aus einer Komponente I und einer Komponente II besteht, daß die Komponente I einen partikelförmigen Träger, der mit Fab- oder F(ab)₂-Fragmenten eines für das nachzuweisende Antigen spezifischen Antikörpers beschichtet ist, und ein Konjugat enthält, das aus wenigstens einem zum Fc-Fragment von Antikörpern reaktiven Protein und einem Indikator besteht, daß die Komponente II für das nachzuweisende Antigen spezifische, monoklonale Antikörper und gegebenenfalls bezüglich des nachzuweisenden Antigens unspezifische IgG-Moleküle bzw. deren Fc-Fragmente enthält.

Diese Mittel können einfach hergestellt werden.

Bevorzugte Ausführungsformen der erfindungsgemäßen Nachweisverfahren, der Reaktionsgemische und der Verfahren zu deren Herstellung sind Gegenstand der Unteransprüche.

Die Entwicklung der klinischen Labordiagnostik schreitet weltweit stark voran und hat damit einen steigenden Bedarf an hochsensitiven, sehr spezifischen und rasch durchzuführenden Testsystemen.

Im Gegensatz zur klinisch-chemischen Massendiagnostik ist vor allem die immunologische uns serologische Diagnostik darauf angewiesen, in kleineren Serien und mit möglichst minimaler Laborausstattung zu arbeiten. Daher zeigt sich hier ein Trend zu geräteunabhängigen Schnelltests.

Im wesentlichen sind derzeit drei Grundsysteme in verschiedenen Varianten verfügbar:
1. Partikelagglutination
2. Immunokonzentration
3. Radial Partition Immunoassay (RPIA).

Bei der Partikelagglutination werden mit Antigen oder Antikörper beschichtete Partikel (meist Latexpartikel oder Erythrozyten) durch eine Immunreaktion zur makroskopisch sichtbaren Agglutination gebracht (direkte Partikelagglutination) oder in der Agglutination gehindert (indirekte oder kompetitive Partikelagglutination).

Diese Testsysteme sind schnell und bedürfen nur einer minimalen Laborausstattung, sind jedoch häufig zu wenig sensitiv und spezifisch und brauchen meist für ihre Auswertung eine gewisse Erfahrung. Darüber hinaus sind sie meist sehr anfällig für störende Einflüsse von Serumkomponenten (Lipide, Rheumafaktoren und dergl.).

Bei der Immunokonzentration werden Antigene oder Antikörper auf definierten Bereichen einer Membran immobilisiert und Probe- und Nachweisreagenzien werden aktiv (über Vakuum) oder passiv (durch Kapillarwirkung) durchgesaugt. (ICON^{(R)} von Hybritech, US-PS Nr. 4.727.109 und 4.632.901). Durch diese Gestaltung der Festphase wird die Kinetik der Antigen-Antikörperinteraktion um den Faktor 10 beschleunigt, sodaß zeitraubende Inkubationsschritte entfallen. Die Detektion erfolgt wie beim Sandwich-ELISA mit enzymmarkierten Sekundärantikörpern und der anschließenden Visualisierung der Enzymreaktion. Es handelt sich dabei um ein mehrstufiges Verfahren, das in 5 bis 10 Minuten Ergebnisse liefert, die in Sensitivität und Spezifität durchaus mit ELISA-Ergebnisssen vergleichbar sind.

Neue Entwicklungen befassen sich auch mit der Verwendung von Latexpartikeln als Antigenträger und deren anschließende Immobilisierung auf diversen Membranen anstatt der Membran-Direktbeschichtungsmethode.

Der Radial Partition Immunoassay vereint die Prinzipien der "solid phase" Immuntechnik mit der radialen Chromatographie. Beim RIPA (radial partition enzyme immunoassay) werden Probe und markiertes Konjugat auf einer festen Matrix (Membran), die immobilisiertes Antigen oder Antikörper enthält, inkubiert. Nach einer geeigneten Inkubationszeit (wenige Minuten) werden die Reaktanten durch einen Waschschritt mittels radialer Chromatographie getrennt. Der zentrale Spot enthält dann nur mehr das Antigen, gebundene Antikörper und gebundenes Konjugat.

Nicht gebundenes Konjugat und Serum bzw. Probenbestandteile diffundieren aus dem Kernbereich der Membran. Die Detektion erfolgt bei der Verwendung von enzymmarkierten Konjugaten durch anschließende Substratzugabe und Visualisierung der Enzymreaktion.

Neuere Nachweisverfahren, basierend auf diesem Prinzip, verwenden als Konjugat Gold markierte Sekundärantikörper, sodaß die Substratzugabe entfällt. Die Vorteile dieser Technologie liegen in der Einfachheit und Schnelligkeit der Durchführung. Unscharfe oder ungenügende Separation der Liganden führt in derartigen Systemen aber immer wieder zu Spezifitätsproblemen oder zu erhöhten Hintergrundreaktionen bei Verwendung enzymgekoppelter Systeme. Außerdem leidet bei Verwendung der radialen Chromatographie mitunter auch die Sensitivität, da vor allem zum Zeitpunkt der Benetzung der Membran (bei Probenauftrag) Reaktanten durch Kapillareffekte aus der Reaktionszone diffundieren können, bevor noch die Immunreaktion stattfinden konnte. Besonders bei Proben mit niedrigem Reaktantengehalt (z.B. schwachtitrige Seren) kann dieser Effekt zu Problemen führen.

Die erfindungsgemäßen Verfahren zum Nachweis von Antikörpern und von Antigenen zeichnen sich dadurch aus, daß sie in einem Schritt alle nötigen Reaktanden zur Reaktion bringen und durch den nachfolgenden einfachen Trennschritt (z.B. Filtration durch eine Membran) einen optisch frei sichtbaren, hoch sensitiven Antikörper- bzw. Antigennachweis gestatten.

Der entscheidende Schritt der Erfindung liegt beim Nachweisen der Antikörper in der Verwendung eines Fc-reaktiven Proteins - konjugiert mit verschiedenen Indikatoren wie Gold, Pigmente, FITC, gefärbte Kunststoffpartikel etc. wie beispielsweise Protein A-Gold-Konjugat, Protein-G-Gold-Konjugat, Anti-Antikörper-Gold-Konjugat, Protein A-Fluorochrome, z.B. FITC-Konjugat, Protein A+G-Farblatex-Konjugat, Farblatex-Komplementfaktor-Konjugat usw. - zur Detektion der Antigen-Antikörperbindung auf den als Träger dienenden Latexpartikeln. Protein A und Protein G sind beispielsweise relativ kleine Proteine, die spezifisch am Fc-Fragment von IgG-Molekülen binden. Der Antikörper wird auch nach der Bindung an Protein A oder Protein G nicht in seiner Antigenbindungskapazität beeinflußt, so daß eine simultane Inkubation beider Reaktanten in flüssiger Phase ermöglicht wird. Darüberhinaus werden beispielsweise durch die Verwendung von Protein A auch spezifische IgM-Antikörper erfaßt, was in vielen Fällen zu einer ganz erheblichen Erhöhung der Sensitivität führt.

Weiters ist für das Antikörper-Nachweis-Verfahren die simultane Inkubation von Detektorreagenz und der zu untersuchenden Probe von zentraler Bedeutung, da in diesem System auch unspezifische IgG Moleküle zur Signalverstärkung genutzt werden. Da auf einem einzigen Goldpartikel bis zu 70 Moleküle Protein A oder Protein G gebunden sind, können diese bis zu 140 IgG Moleküle binden, welche ihrerseits wiederum an auf anderen Goldpartikeln gebundenen Protein A oder -G Moleküle binden können. Die dabei entstehenden Komplexe können, auch wenn nur ein einziges IgG Molekül aus dem Komplex ein spezifischer Antikörper ist, von den Latexpartikeln, auf denen Antigen immobilisiert ist, spezifisch gebunden und anschließend auf der Membran zurückgehalten werden. Das Signal wird dabei durch das Mitwirken der nicht antigenspezifischen Antikörper um ein Vielfaches verstärkt.

Sämtliche immunologische Reaktionen (Antigen-Antikörperbindung und Detektion) erfolgen bei der Erfindung gleichzeitig und oft innerhalb von nur 60 Sekunden. Im darauffolgenden Trennschritt (Filtration) erfolgt zum zweiten dann die vollständige Abtrennung überflüssiger Reaktanten. Unspezifische Reaktionen werden in einem einzigen Waschschritt in Anschluß an die Filtration eliminiert. Das Ergebnis ist sofort, also ohne Substratzugabe, sichtbar und es bedarf auch keines Abstoppens irgendwelcher Enzymreaktionen. Durch die wenigen und sehr einfach durchzuführenden Reaktionsschritte werden darüberhinaus Fehlerquellen bei der Testdurchführung weitestgehend eliminiert.

Die Verwendung von Latexpartikeln als Antigenträger in flüssiger Phase ermöglicht eine optimale Beschleunigung der Kinetik der Antigen-Antikörperinteraktion, da durch die Suspension der Latexpartikel diffusionsbedingte Prozeßlimitationen vermieden werden können. Zusätzlich beschleunigt wird diese Reaktion durch die durch die Gravitation bedingte Sedimentation der Latexpartikel während der Inkubation, so daß eine permanente Bewegung (Schütteln) des Testansatzes entfallen kann.

Die Testdurchführung ist äußerst einfach und besteht lediglich aus einer zu untersuchenden Probelösung (z.B. Serumverdünnung), Zugabe dieser zum Reaktionsgemisch, Überführen des Reaktionsgemisches auf die Filtervorrichtung und vor oder nach erfolgter Filtration Zugabe von fünf Tropfen Waschlösung (zum Wegwaschen überflüssiger Reaktanden). Die Waschlösung kann auch bereits unmittelbar vor dem Filtrieren zugegeben werden, so daß das Ergebnis unmittelbar nach dem Filtrieren ablesbar ist. Eine Zubereitung der Reagenzien entfällt. Der Zeitbedarf für den kompletten Nachweis beträgt höchstens 3 Minuten. Der Nachweis eignet sich sowohl zur Durchführung von Einzeltests als auch für die Prüfung größerer bis großer Serien, da eine Automatisation unter Verwendung der gängigsten ELISA-Prozessoren sehr leicht möglich ist. Da die Intensität der Farbreaktion über einen weiten Bereich proportional zum Antikörpergehalt der Probe ist, ist bei Verwendung von Dot-Scannern auch eine semi-quantitative bzw. quantitative Auswertung möglich.

Das erfindungsgemäße Verfahren zum Nachweis von Antigenen besitzt dem Vorteil, daß in einem Schritt alle nötigen Reaktanden zur Reaktion gebracht werden und daß es durch die nachfolgende einfache Trennung (z.B. Filtration durch eine Membran) einen optisch frei sichtbaren, hoch sensitiven Antigennachweis gestattet.

Ein Merkmal dieses Verfahrens zum Nachweisen von Antigenen liegt in der Verwendung eines Fc-reaktiven Proteins - konjugiert mit verschiedenen Indikatoren wie Gold, Pigmente, Fluorochrome, gefärbte Kunststoffpartikel etc. wie beispielsweise Protein A-Gold Konjugat, Protein-G-Gold Konjugat, Anti-Anitkörper Gold Kojugat, Protein A FITC, Protein A+G Farblatex Konjugat, Komplementfaktoren etc. - zur Detektion der Antigen-Fab - oder F(ab)₂-Fragment-Bindung auf den Latexpartikeln. Protein A und Protein G sind beispielsweise relativ kleine Proteine, die spezifisch am Fc-Fragment von IgG-Molekülen binden. Der Antikörper wird auch nach der Bindung an Protein A oder Protein G nicht in seiner Antigenbindungskapazität beeinflußt, sodaß eine simultane Inkubation beider Reaktanten in flüssiger Phase möglich wird.

Weiters ist für das Verfahren zum Nachweisen von Antigenen die simultane Inkubation von Detektorreagenz und der zu untersuchenden Probe von zentraler Bedeutung, da bei der Erfindung auch unspezifische IgG Moleküle zur Signalverstärkung genutzt werden. Da auf einem einzigen Goldpartikel bis zu 70 Moleküle Protein A oder Protein G gebunden sind, können diese bis zu 140 IgG Moleküle binden, die ihrerseits wiederum an auf anderen Goldpartikeln gebundenen Protein A oder -G Moleküle binden können. Die dabei entstehenden Komplexe können dann, auch wenn nur ein einziger Antikörper aus dem Komplex ein spezifischer Antikörper ist, von den Latexpartikeln spezifisch gebunden und anschließend auf der Membran zurückgehalten werden. Das Signal wird dabei durch das Mitnehmen der nicht antigenspezifischen Antikörpern um ein Vielfaches verstärkt.

Sämtliche immunologische Reaktionen (Antigen-Fab- bzw. F(ab)₂-Fragment-Bindung und Detektion) erfolgen bei der Erfindung gleichzeitig und oft innerhalb von nur 60 Sekunden. Im darauffolgenden Trennschritt (Filtration) erfolgt die vollständige Abtrennung überflüssiger Reaktanten. Unspezifische Reaktionen werden in einem einzigen Waschschritt in Anschluß an die Trennung (Filtration) eliminiert. Das Ergebnis ist sofort, also ohne Substratzugabe, sichtbar und es bedarf auch keines Abstoppens irgendwelcher Enzymreaktionen. Durch die wenigen und sehr einfach durchzuführenden Reaktionsschritte werden darüberhinaus Fehlerquellen beim Durchführen des erfindungsgemäßen Verfahrens weitestgehend eliminiert.

Die Verwendung von Latexpartikeln als Träger für die Fab- oder F(ab)₂-Fragmente in flüssiger Phase ermöglichen eine optimale Beschleunigung der Kinetik der Antigen-Antikörper-Reaktion, da durch die Suspension der Latexpartikel diffusionsbedingte Prozeßlimitationen vermieden werden. Zusätzlich beschleunigt wird diese Reaktion durch die durch die Gravitation bedingte Sedimentation der Latexpartikel während der Inkubation, sodaß eine permanente Bewegung (Schütteln) des Testansatzes entfallen kann.

Die Testdurchführung beim Nachweisen von Antigenen ist äußerst einfach und besteht lediglich aus einer zu untersuchenden Probelösung (z.B. Serumverdünnung), Zugabe dieser zu der einen Komponente des Mittels mit nachfolgender Zugabe der zweiten Komponente, Überführen des Reaktionsgemisches auf die Filtervorrichtung und nach erfolgter Filtration Zugabe von 5 Tropfen Waschlösung (zum Wegwaschen überflüssiger Reaktanden). Die Waschlösung kann auch schon vor der Filtration zugesetzt werden. Ein Zubereiten der Reagenzien entfällt. Der Zeitbedarf für den vollständigen Nachweis beträgt höchstens 3 Minuten. Das Verfahren eignet sich sowohl zum Durchführen von Einzeltests als auch für größere bis große Serien, da eine Automatisation unter Verwendung der gängigsten ELISA-Prozessoren sehr leicht möglich ist.

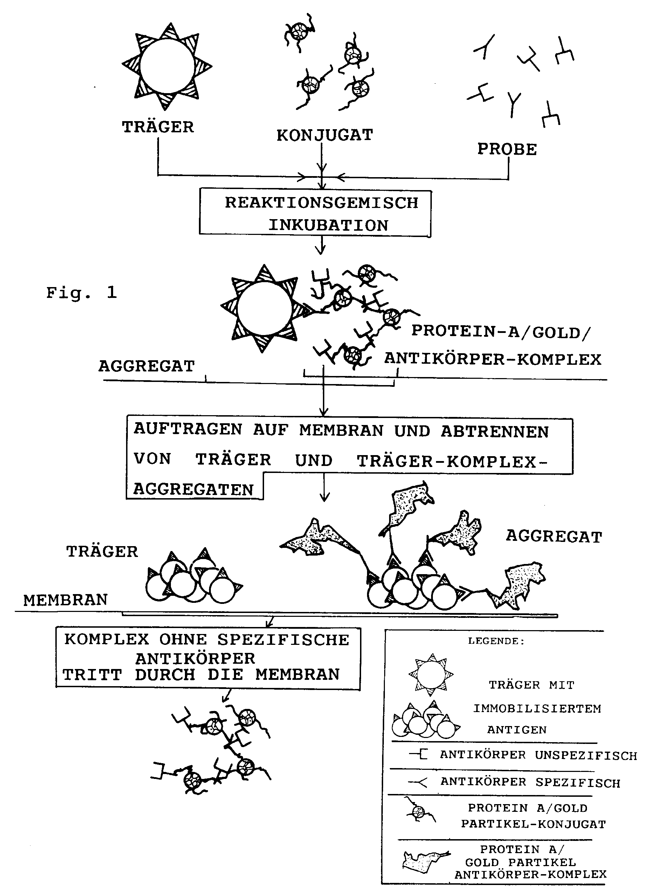

In den angeschlossenen Zeichnungen ist in
Fig. 1 das Verfahren der Erfindung zum Nachweisen von Antikörpern, in
Fig. 2 das Nachweisverfahren der Erfindung für Antigene und in
Fig. 3 die Herstellung von Fab-, F(ab)₂- und Fc- Fragmenten jeweils schematisch und beispielhaft dargestellt.

Das Verfahren der Erfindung eignet sich für alle Nachweisverfahren, in denen Antigen-Antikörperreaktionen als wesentlicher Bestandteil des Systems stattfinden, wie beispielsweise alle serologischen Nachweise von Virus- oder Bakterien-Infektionen in Lebewesen (sowohl Mensch als auch Tier), die spezifische Antikörper induzieren.

Nachstehend werden Beispiele für das Herstellen des erfindungsgemäßen Reaktionsgemisches und für das erfindungsgemäße Verfahren zum Nachweis von Antikörpern angegeben.

### Beispiel 1:

a) Gewaschene, uniforme Latexpartikel (Polystyrol) mit einem durchschnittlichen Durchmesser von 1 µm werden in alkalischem Beschichtungspuffer, in dem rekombinanten Membranantigen (gp41-HIV-1) in Konzentrationen zwischen 200 und 800 µg/ml sowie rekombinantes Hüll-Antigen (p24 HIV-1) in Konzentrationen zwischen 50 und 200 µg/ml gelöst sind, beschichtet und anschließend gewaschen. Freie Proteinbindungsstellen werden mit einem Gemisch aus Trägerproteinen und Detergentien abgesättigt. Anschließend werden die beschichteten Partikel erneut gewaschen und in einer physiologischen Pufferlösung resuspendiert. Für das Herstellen des fertigen Reaktionsgemisches wird dieser Suspension Protein-A-Gold Konjugat und/oder Protein-G-Gold Konjugat (jeweils 20 nm Gold Partikel gekoppelt an die Proteine A₅₂₅ = 4.0) im Verhältnis 16 : 1 zugegeben. Dieses Reaktionsgemisch stellt die zentrale Testkomponente dar und kann in beliebigen Mengen bei 2 bis 8°C stabil gelagert werden.
b) Für die Testdurchführung wird Patientenserum je nach Anwendung zwischen 1:10 und 1:50 verdünnt. Von der entsprechenden Verdünnung werden 40 µl mit 160 µl Reaktionsgemisch vermischt und 2 bis 5 Minuten inkubiert.
c) Danach wird diese Suspension aus b) auf eine Zelluloseacetatmembran oder eine Nylonmembran, unter der sich ein saugfähiges Kissen oder Vakuum befindet, transferiert. Die Trennung der flüssigen von der festen Phase erfolgt dabei spontan.
d) Es wird mit 5 Tropfen Detergens-Puffer nachgewaschen (oder die Waschlösung gleich dem Reaktionsgemisch zugesetzt). Ein je nach spezifischem Antikörpergehalt der Probe mehr oder weniger intensiv rot gefärbter Kreis zeigt HIV-1-Positivität des Serums an. Bei negativen Reaktionen bleibt die Membran weiß.

Für alle nachfolgend genannten Beispiele bleiben die wesentlichen Schritte des im Beispiel 1 beschriebenen Verfahrens gleich. Es werden daher nur mehr die Änderungen beschrieben.

### Beispiel 2:

Als Antigene werden rekombinantes Antigen gp 36 HIV-2 und p 26 HIV-2 für die Beschichtung der Latexpartikel verwendet. Eine Farbreaktion zeigt HIV-2-Positivität des Serums an.

### Beispiel 3:

Für die Beschichtung der Latexpartikel wird ein Gemisch aus HIV-1 und HIV-2 rekombinanten Antigenen verwendet. Eine Farbreaktion zeigt HIV-1 oder HIV-2 Positivität des Serums an.

### Beispiel 4:

Für die Beschichtung der Latexpartikel wird Membranantigen aus Entamoeba histolytica verwendet. Eine Farbreaktion zeigt eine Infektion mit dem Erreger der Amoebenruhr an.

### Beispiel 5:

Für die Latexbeschichtung wird eine Antigenpräparation aus dem Rinderherpesvirus IBR/IPV verwendet. Als Detektorreagenz wird ausschließlich Protein-G-Gold verwendet. Für die Prüfung wird Rinderserum oder -Plasma bzw. Milch verwendet. Eine Farbreaktion zeigt das Vorliegen einer IBR/IPV Infektion beim getesteten Rind an.

### Beispiel 6:

Für die Latexbeschichtung wird eine Antigenpräparation aus dem Felinen Leukämievirus verwendet. Für die Untersuchung werden Katzenserum oder -plasma verwendet. Eine Farbreaktion zeigt das Vorliegen einer FeLV-Infektion bei der untersuchten Katze an.

### Beispiel 7:

Für die Latexbeschichtung wird eine Antigenpräparation aus dem Parvo Virus beim Schwein verwendet. Für die Untersuchung werden Schweineserum oder -plasma verwendet. Eine Farbreaktion zeigt das Vorliegen einer Parvovirusinfektion des untersuchten Schweines an.

### Beispiel 8:

Für die Latexbeschichtung wird eine Antigenpräparation von Salmonella pulorum verwendet. Für die Untersuchung wird Hühnerserum oder -plasma verwendet. Eine Farbreaktion zeigt das Vorliegen einer Salmonelleninfektion des untersuchten Huhnes an.

### Beispiel 9:

Für die Latexbeschichtung wird eine Antigenpräparation aus Salmonella typhii verwendet. Für die Untersuchung wird Hühnerserum pder -plasma verwendet. Eine Farbreaktion zeigt das Vorliegen einer Infektion mit Salmonella typhii an.

### Beispiel 10:

Für die Latexbeschichtung wird rekombinantes Hepatitis B-Virus Antigen (HBV) verwendet. Für die Untersuchung wird humanes Serum oder Plasma verwendet. Für die Untersuchung wird humanes Serum oder Plasma verwendet. Eine Farbreaktion zeigt das Vorliegen einer HBV-Infektion an.

### Beispiel 11:

Für die Latexbeschichtung wird eine Antigenpräparation aus dem Epstein Barr Virus (EBV) verwendet. Für die Untersuchung wird humanes Serum oder Plasma verwendet. Eine Farbreaktion zeigt das Vorliegen einer EBV-Infektion an.

### Beispiel 12:

Für die Latexbeschichtung wird rekombinantes Membranantigen des Maedi Visna Virus (MVV) verwendet. Für die Untersuchung werden Schafserum oder -plasma verwendet. Eine Farbreaktion zeigt das Vorliegen einer MVV-Infektion des untersuchten Schafes an.

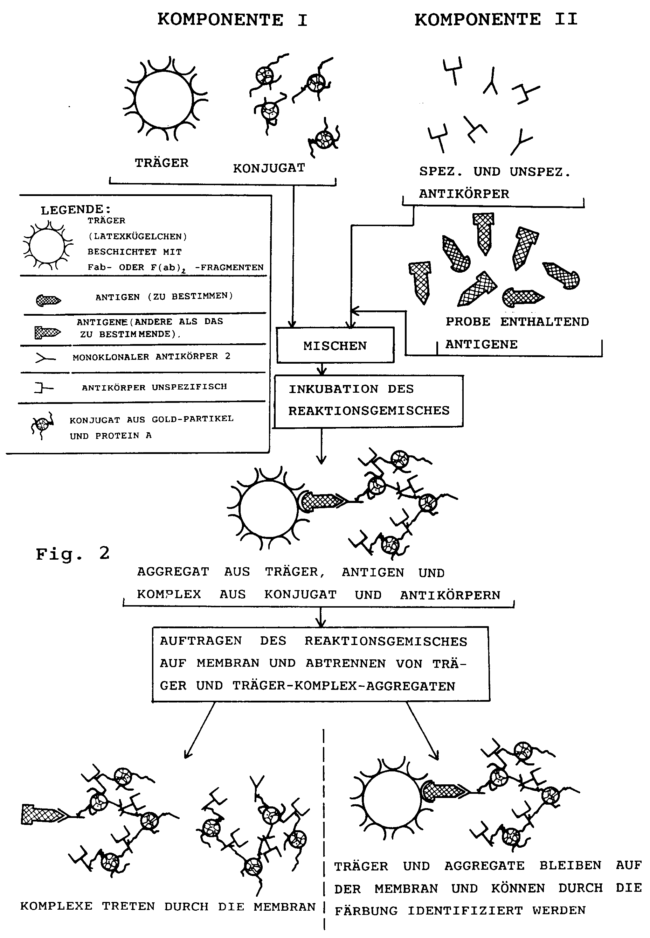

Bei dem Verfahren und dem Testsystem für den Nachweis von Antigenen werden auf den Latexpartikeln Fab- oder F(ab)₂-Fragmente eines für das zu detektierende Antigen spezifischen monoklonalen Antikörpers (Antikörper 1) immobilisiert (vgl. Fig. 2). Zusätzlich wird im Reaktionsgemisch ein weiterer monoklonaler Antikörper vorgelegt, der sich in seiner Epitopspezifität vom immobilisierten Antikörper unterscheidet.

Bei dem Nachweis von Antigen(en) erfolgen sämtliche Reaktionsschritte simultan innerhalb von beispielsweise 60 Sekunden. Die Detektion erfolgt unter Verwendung von Fc-reaktiven Protein-Gold-, Protein-Farblatex- oder Protein-Farbpartikel-Konjugaten (wie z.B. Protein-A-Gold, Protein-G-Gold, Protein A-Farblatex). Bei der Verwendung von IgG-hältigen Antigenproben (Serum, Plasma) werden unspezifische IgG-Moleküle zur Signalverstärkung genützt. Nicht oder nur geringfügig IgG-hältige Antigenproben (Urin) können durch Zugabe von Fc-Fragmenten oder gereinigtem IgG zum Reaktionsgemisch ebenfalls vom erfindungsgemäßen Verstärkungsmechanismus profitieren.

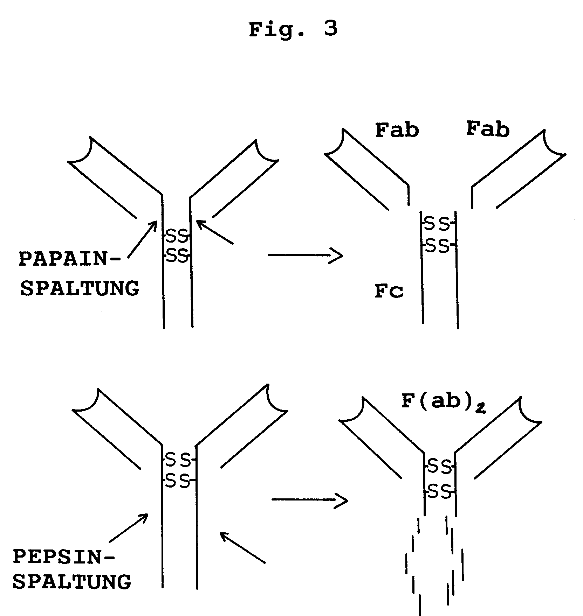

Fc-, Fab- bzw. F(ab)₂-Fragmente von Immunglobulinen können durch Spaltung von IgG mit Papain (Fc und Fab) bzw. Pepsin (Fc und F(ab)₂) und anschließender chromatographischer Trennung der Fragmente erhalten werden (Fig. 3).

Für das Herstellen des Reaktionsgemisches ist zu beachten, daß dieses aus zwei Komponenten besteht, von denen eine unmittelbar vor Verwendung mit der Probe versetzt wird und das so erhaltene Gemisch mit der anderen Komponente gemischt wird. Idealerweise besteht eine Komponente (Komponente I) aus mit Fab-Fragmenten beschichteten Latexpartikeln mit dem Detektoreagenz (z.B. Protein-A-Gold) in einer physiologischen Pufferlösung, die andere Komponente (Komponente II) aus dem antigenspezifischen monoklonalen Antikörper und (gegebenenfalls) unspezifischen IgG-Molekülen bzw. deren Fc-Fragmenten ebenfalls in einer physiologischen Pufferlösung. Unspezifische IgG-Moleküle bzw. deren Fc-Fragmente werden nur verwendet, wenn das Verfahren zum Nachweis von Antigenen in nicht IgG-hältigen Flüssigkeiten (Proben) verwendet wird.

Die Testdurchführung (Nachweis) erfolgt dann wie folgt:

Zu einer Verdünnung der zu bestimmenden Antigenlösung wird zuerst Komponente II zugegeben und unmittelbar darauf mit Komponente I vermengt. Die weiteren Schritte sind Inkubieren, Trennung (Filtration) und das Auswerten.

Durch entsprechendes Einstellen der einzelnen Testkomponenten kann jene Kombination ermittelt werden, bei deren Verwendung eine (lineare) Korrelation zwischen Signalintensität und Antigengehalt einer Probe besteht. Bei Verwendung von geeigneten Auswertesystemen (Dot-Scannern) wird bei gleichzeitiger Bestimmung von Antigen-Standardproben bekannter Konzentration auch eine quantitative Antigenbestimmung ermöglicht.

Im einzelnen kann beim Nachweis von Antigen unter Anwendung des Verfahrens gemäß der Erfindung beispielsweise wie folgt vorgegangen werden:

### 1.) Herstellen der Komponente I des Reaktionssystems:

Aus für das zu detektierende Antigen spezifischen, monoklonalen Antikörpern (Antikörper 1) werden entweder durch Papainspaltung Fab- Fragmente oder durch Pepsinspaltung F(ab)₂-Fragmente (siehe Fig. 3) hergestellt. Mit den so gewonnenen Fab-oder F(ab)₂-Fragmenten werden Träger, z.B. Latexpartikel (aus Polystyrol) mit einem Durchmesser von 1 µm beschichtet.

Weiters wird ein Konjugat aus Fc-reaktivem Protein (Protein A) und einem Indikator (z.B. Goldpartikel, FITC oder Farblatex) hergestellt (beispielsweise Protein-A-Gold-, Protein-G-Gold-, Protein-A-Farblatex-Konjugat).

Der beschichtete Träger und das Konjugat werden in eine Pufferlösung, insbesondere eine physiologische Pufferlösung, eingetragen und die so hergestellte Komponente I für die Bestimmung vorrätig gehalten.

### 2.) Herstellen der Komponente II des Reaktionssystems:

Bezüglich des zu detektierenden Antigens spezifische, monoklonale Antikörper (Antikörper 2) und bezüglich des zu detektierenden Antigens unspezifische IgG-Moleküle (Antikörper) bzw. deren Fc-Fragmente werden ebenfalls in eine physiologische Pufferlösung eingetragen.

Unspezifische IgG-Moleküle bzw. deren Fc-Fragmente werden in die Komponente II nur eingebracht, wenn eine IgG-arme oder IgG-freie Probe (z.B. Urin), nicht aber eingebracht, wenn eine ohnedies IgG-haltige Antigenprobe (Serum, Plasma) untersucht wird.

### 3.) Durchführen des Nachweisverfahrens:

Unmittelbar vor dem Nachweis wird in die Komponente II die mit einer Pufferlösung, insbesondere mit einer physiologischen Pufferlösung, verdünnte Lösung von Antigenen (Probe, die allenfalls das nachzuweisende Antigen enthält) zugegeben. Dabei wird eine Komponente II mit unspezifischen IgG-Molekülen (Antikörpern) oder deren Fc-Fragmenten angewendet, wenn in der Antigen(e) enthaltenden Probe (z.B. Urin) wenig oder keine IgG-Moleküle enthalten sind.

Wenn eine IgG-haltige Probe (z.B. Plasma, Serum) dem Nachweisverfahren unterworfen wird, wird die mit Pufferlösung (physiologische Pufferlösung) verdünnte, zu bestimmende Antigenlösung in eine Komponente II eingetragen, die nicht mit unspezifischen IgG-Molekülen (Antikörpern) oder deren Fc-Fragmenten versetzt wurde. Dem so erhaltenen Gemisch wird die Komponente I zugegeben.

Das so erhaltene Reaktionsgemisch wird 2 bis 5 min, oder 30 bis 120 sek lang, insbesondere 2 min oder 60 sek lang bei einer Temperatur von 19 bis 30°C inkubiert. Bei der Reaktion bilden sich Komplexe aus Konjugat und den entweder in Komponente II oder in der Probe enthaltenen Antikörpern (Antikörper 2 und IgG-Moleküle). Die Komplexe reagieren über die in ihnen enthaltenen, bezüglich des nachzuweisenden Antigens spezifischen Antikörper 2 mit dem Antigen. Das nachzuweisende Antigen bindet sich seinerseits an die Fab- oder F(ab)₂-Fragmente (des Antikörpers 1), mit welchen der Träger beschichtet ist. So entstehen Aggregate aus Träger, zu bestimmendem Antigen und Komplexen aus Konjugat und Antikörpern 2.

Nach dem Inkubieren wird über eine Membran (z.B. eine Nylonmembran oder eine Zelluloseacetatmembran) filtriert, wobei Komplexe ohne angelagerte Antigene und Kom-plexe mit angelagerten, nicht nachzuweisenden Antigenen durch die Membran hindurchtreten, wogegen die oben beschriebenen Aggregate, die nachzuweisende Antigene enthalten, auf der Membran zurückgehalten werden.

Im Anschluß an den Filtrationsvorgang wird mit einer Detergens-Pufferlösung gewaschen, oder es wird die Wschlösung dem Reaktionsgemisch unmittelbar zugesetzt.

Das von den auf der Membran zurückgehaltenen Aggregaten gebildete Signal (Färbung, bei Verwendung von Goldpartikeln als Indikator eine Rotfärbung) dient als Signal für die Anwesenheit von nachzuweisendem Antigen, d.h. einem solchen, für welches das auf dem Träger immobilisierte Fab- oder F(ab)₂-Fragment spezifisch ist.

Durch gezielte Kombination von Komponente I und Komponente II liegt eine Korrelation zwischen Signalintensität und Antigengehalt der Probe vor. Es ist daher mit Hilfe von Auswertesystemen (Dot-Scannern) und gleichzeitiger Bestimmung von Antigenstandardproben bekannter Konzentration eine quantitative Antigenbestimmung möglich.

### Beispiele für Nachweisverfahren von Antigenen:

### Beispiel 13:

Bestimmung von hCG im Urin oder Serum unter Verwendung von zwei monoklonalen anti-hCG Antikörpern zur Feststellung von Schwangerschaften oder für die Tumordiagnostik.

### Beispiel 14:

Bestimmung von HIV-1 p24 Antigenen unter Verwendung von zwei monoklonalen anti-p24 Antikörpern zum HIV-Antigennachweis.

### Beispiel 15:

Bestimmung von Hepatitis B-Antigenen im Serum oder im Plasma unter Verwendung von zwei monoklonalen anti-Hepatitis-B-Antikörpern.

### Beispiel 16:

Bestimmung von PMSG unter Verwendung von zwei monoklonalen anti-PMSG-Antikörpern zur Ermittlung der Trächtigkeit von Stuten.

### Beispiel 17:

Bestimmung von Mykotoxinen (Aflatoxin, Zearalenon ...) in Lebens- bzw. Futtermitteln unter Verwendung von jeweils zwei spezifischen monoklonalen Antikörpern.

### Beispiel 18:

Bestimmung diverser Tumormarker (z.B. α-Fetoprotein) unter Verwendung von zwei spezifischen monoklonalen Antikörpern.

Zusammenfassend kann die Erfindung beispielsweise wie folgt dargestellt werden:

Zum Nachweisen eines in einer Probe enthaltenen Antikörpers wird die Probe in ein Reaktionsgemisch, das einen partikelförmigen Träger, an den ein bezüglich des nachzuweisenden Antikörpers spezifisches Antigen, wie beispielsweise native oder rekombinante Virus-Antigene, native oder rekombinante Bakterien-Antigene, native oder rekombinante Pilz-Antigene oder sonstige native oder rekombinante zellspezifische Antigene immobilisiert sind, und ein Konjugat aus wenigstens einem zum Fc-Fragment von Antikörpern reaktiven Protein und einem an dieses gebundenen Indikator enthält, eingetragen. Hierauf wird inkubiert, um Komplexe aus Konjugat und Antikörpern zu bilden und gleichzeitig diese Komplexe an den Träger zu binden. Hierauf werden der Träger und Aggregate aus Träger und wenigstens einen nachzuweisenden Antikörper enthaltenden Komplexen vom flüssigen Reaktionsgemisch und von Komplexen, die ausschließlich nicht nachzuweisende Antikörper enthalten, abgetrennt, und ohne zusätzliche immunchemische Reaktion auf das Vorhandensein von an den Träger gebundenen Komplexen aus Konjugat und dem nachzuweisenden Antikörper geprüft.

Ein in einer Probe enthaltenes Antigen kann durch Binden des nachzuweisenden Antigens an ein Fab- oder F(ab)₂-Fragment eines für das nachzuweisende Antigen spezifischen, monoklonalen Antikörpers nachgewiesen werden. Hiezu wird die Probe in einem Reaktionsgemisch, das einen partikelförmigen Träger, an dem Fab- oder F(ab)₂-Fragmente eines für das nachzuweisende Antigen spezifischen monoklonalen Antikörpers immobilisiert sind, und ein Konjugat, das aus wenigstens einem zum Fc-Fragment von Antikörpern reaktiven Protein und einem an dieses gebundenen Indikator besteht, sowie wenigstens einen bezüglich des nachzuweisenden Antigens spezifischen monoklonalen Antikörper enthält, inkubiert. Dabei bilden sich Aggregate aus partikelförmigem Träger, dem nachzuweisenden Antigen und Komplexen bestehend aus Konjugat und Antikörpern. Das Reaktionsgemisch wird filtriert und auf dem Filter auf die Anwesenheit (Farbreaktion) von das nachzuweisende Antigen enthaltenden Aggregaten geprüft.

Ein Mittel zum Nachweisen eines in einer Probe enthaltenen Antigens, das zur Anwendung beim Durchführen des Verfahrens geeignet ist, besteht aus einer Komponente I und einer Komponente II. Die Komponente I enthält einen partikelförmigen Träger, der mit Fab- oder F(ab)₂-Fragmenten eines für das nachzuweisende Antigen spezifischen Antikörpers beschichtet ist, und ein Konjugat, das aus wenigstens einem zum Fc-Fragment von Antikörpern reaktiven Protein und einem Indikator besteht. Die Komponente II enthält für das nachzuweisende Antigen spezifische, monoklonale Antikörper und gegebenenfalls bezüglich des nachzuweisenden Antigens unspezifische IgG-Moleküle bzw. deren Fc-Fragmente.

## Patentansprüche

1. Verfahren zum Nachweisen eines in einer Probe enthaltenen Antikörpers durch Binden des nachzuweisenden Antikörpers an ein bezüglich des nachzuweisenden Antikörpers spezifisches Antigen, dadurch gekennzeichnet, daß man die Probe in ein Reaktionsgemisch, das einen partikelförmigen Träger, an den ein bezüglich des nachzuweisenden Antikörpers spezifisches Antigen immobilisiert ist, und ein Konjugat aus wenigstens einem zum Fc-Fragment von Antikörpern reaktiven Protein und einem an dieses gebundenen Indikator enthält, einträgt und inkubiert, um Komplexe aus Konjugat und Antikörpern zu bilden und diese Komplexe an den Träger zu binden, daß man hierauf den Träger abtrennt, und daß man auf das Vorhandensein von an den Träger gebundenen Komplexen aus Konjugat und dem nachzuweisenden Antikörper prüft.

2. Verfahren zum Nachweisen eines in einer Probe enthaltenen Antigens durch Binden des nachzuweisenden Antigen an ein Fab- oder F(ab)₂-Fragment eines für das nachzuweisende Antigen spezifischen, monoklonalen Antikörpers, dadurch gekennzeichnet, daß man die Probe in einem Reaktionsgemisch, das einen partikelförmigen Träger, an dem Fab- oder F(ab)₂-Fragmente eines für das nachzuweisende Antigen spezifischen monoklonalen Antikörpers immobilisiert sind, und ein Konjugat, das aus wenigstens einem zum Fc-Fragment von Antikörpern reaktiven Protein und einem an dieses gebundenen Indikator besteht, sowie wenigstens einen bezüglich des nachzuweisenden Antigens spezifischen monoklonalen Antikörper, der sich in seiner Epitopspezifität vom immobilisiererten Antikörper unterscheidet, enthält, inkubiert, um Aggregate aus partikelförmigem Träger, dem nachzuweisenden Antigen und Komplexen bestehend aus Konjugat und Antikörpern zu bilden, und daß man auf die Anwesenheit von Aggregaten Prüft, die das nachzuweisende Antigen enthalten.

3. Verfahren nach Anspruch 1 oder 2, dadurch gekennzeichnet, daß man als Träger Latexpartikel, insbesondere solche aus Polystyrol verwendet.

4. Verfahren nach Anspruch 3, dadurch gekennzeichnet, daß man Latexpartikel mit einem durchschnittlichen Durchmesser von 1 µm verwendet.

5. Verfahren nach einem der Ansprüche 1 bis 4, dadurch gekennzeichnet, daß man als Indikator Gold, Pigmente, FITC, gefärbte Kunststoffteilchen verwendet.

6. Verfahren nach einem der Ansprüche 1 bis 5, dadurch gekennzeichnet, daß man ein Reaktionsgemisch verwendet, das als Konjugat Protein-A-Gold Konjugat, Protein-G-Gold Konjugat, Anti-Antikörper-Gold-Konjugat, Fc-reaktives Protein-Fluorochrom-Konjugat oder Protein-A+G-Farblatex-Konjugat enthält.

7. Verfahren nach Anspruch 6, dadurch gekennzeichnet, daß man ein Reaktionsgemisch verwendet, das Konjugat Protein-A-Gold-Konjugat und/oder Protein-G-Gold-Konjugat ist, und daS die Goldpartikel einen mittleren Durchmesser von 20 nm aufweisen.

8. Verfahren nach einem der Ansprüche 1 bis 7, dadurch gekennzeichnet, daß man ein Reaktionsgemisch verwendet, das die Reaktionspartner in einer Pufferlösung, insbesondere einer physiologischen Pufferlösung, enthält.

9. Verfahren nach einem der Ansprüche 1 bis 8, dadurch gekennzeichnet, daß man den Träger durch Filtration abtrennt.

10. Verfahren nach Anspruch 9, dadurch gekennzeichnet, daß man den Träger durch Filtration unter Verwendung einer Membran, insbesondere einer Zelluloseacetatmembran abtrennt.

11. Verfahren nach Anspruch 9 oder 10, dadurch gekennzeichnet, daß man die Filtration durch Anwenden von Vakuum oder durch Anordnen eines saugfähigen Kissens unter dem Filter, insbesondere der Membran unterstützt.

12. Verfahren nach einem der Ansprüche 1 bis 11, dadurch gekennzeichnet, daß man nach der Zugabe der Probe zu dem Reaktionsgemisch 2 bis 5 min lang, vorzugsweise 2 min lang bei einer Temperatur von 19 bis 30°C inkubiert.

13. Verfahren nach einem der Ansprüche 1 bis 12, dadurch gekennzeichnet, daß man den abgetrennten Träger wäscht, wobei man die Waschflüssigkeit dem Reaktionsgemisch bevorzugt bereits vor oder während des Abtrennens des Trägers zusetzt.

14. Verfahren nach Anspruch 13, dadurch gekennzeichnet, daß man mit Hilfe einer Detergens-Pufferlösung wäscht.

15. Verfahren nach einem der Ansprüche 1 und 3 bis 14, dadurch gekennzeichnet, daß man einen Träger verwendet, an dem ein natives oder rekombinantes Virus-Antigen immobilisiert ist.

16. Verfahren nach einem der Ansprüche 1 und 3 bis 14, dadurch gekennzeichnet, daß man einen Träger verwendet, an dem ein natives oder rekombinantes Bakterien-Antigen immobilisiert ist.

17. Verfahren nach einem der Ansprüche 1 und 3 bis 14, dadurch gekennzeichnet, daß man einen Träger verwendet, an dem ein natives oder rekombinantes Pilz-Antigen immobilisiert ist.

18. Verfahren nach einem der Ansprüche 1 und 3 bis 14, dadurch gekennzeichnet, daß man einen Träger verwendet, an dem ein natives oder rekombinantes zellspezifisches Antigen immobilisiert ist.

19. Verfahren nach einem der Ansprüche 1 und 3 bis 14, dadurch gekennzeichnet, daß man einen Träger verwendet, an dem ein rekombinantes, zellspezifisches Antigen immobilisiert ist.

20. Verfahren nach einem der Ansprüche 1 und 3 bis 14, dadurch gekennzeichnet, daß man ein Reaktionsgemisch verwendet, das einen partikelförmigen Träger enthält, der mit rekombinantem Membranantigen (gp41-HIV-1) und mit rekombinantem Hüll-Antigen (p24 HIV-1) beschichtet ist.

21. Verfahren nach einem der Ansprüche 1 und 3 bis 14, dadurch gekennzeichnet, daß man ein Reaktionsgemisch verwendet, das einen partikelförmigen Träger enthält, der mit rekombinantem Antigen gp 36 HIV-2 und p 26 HIV-2 beschichtet ist.

22. Verfahren nach einem der Ansprüche 1 und 3 bis 14, dadurch gekennzeichnet, daß man ein Reaktionsgemisch verwendet, das einen partikelförmigen Träger enthält, der mit einem Gemisch aus HIV-1 und HIV-2 rekombinanten Antigenen beschichtet ist.

23. Verfahren nach einem der Ansprüche 1 und 3 bis 14, dadurch gekennzeichnet, daß man ein Reaktionsgemisch verwendet, das einen partikelförmigen Träger enthält, der mit Membranantigen aus Entamoeba histolytica beschichtet ist.

24. Verfahren nach einem der Ansprüche 1 und 3 bis 14, dadurch gekennzeichnet, daß man ein Reaktionsgemisch verwendet, das einen partikelförmigen Träger enthält, der mit einer Antigenpräparation aus Rinderherpesvirus IBR/IPV beschichtet ist, und das als Konjugat Protein-G-Gold-Konjugat enthält.

25. Verfahren nach einem der Ansprüche 1 und 3 bis 14, dadurch gekennzeichnet, daß man ein Reaktionsgemisch verwendet, das einen partikelförmigen Träger enthält, der mit einer Antigenpräparation aus dem Felinen Leukämievirus beschichtet ist.

26. Verfahren nach einem der Ansprüche 1 und 3 bis 14, dadurch gekennzeichnet, daß man ein Reaktionsgemisch verwendet, das einen partikelförmigen Träger enthält, der mit einer Antigenpräparation aus dem Parvo Virus beim Schwein beschichtet ist.

27. Verfahren nach einem der Ansprüche 1 und 3 bis 14, dadurch gekennzeichnet, daß man ein Reaktionsgemisch verwendet, das einen partikelförmigen Träger enthält, der mit einer Antigenpräparation von Salmonella pulorum beschichtet ist.

28. Verfahren nach einem der Ansprüche 1 und 3 bis 14, dadurch gekennzeichnet, daß man ein Reaktionsgemisch verwendet, das einen partikelförmigen Träger enthält, der mit einer Antigenpräparation aus Salmonella typhii beschichtet ist.

29. Verfahren nach einem der Ansprüche 1 und 3 bis 14, dadurch gekennzeichnet, daß man ein Reaktionsgemisch verwendet, das einen partikelförmigen Träger enthält, der mit rekombinantem Hepatitis B-Virus Antigen (HBV) beschichtet ist.

30. Verfahren nach einem der Ansprüche 1 und 3 bis 14, dadurch gekennzeichnet, daß man ein Reaktionsgemisch verwendet, das einen partikelförmigen Träger enthält, der mit einer Antigenpräparation aus dem Epstein Barr Virus (EBV) beschichtet ist.

31. Verfahren nach einem der Ansprüche 1 und 3 bis 14, dadurch gekennzeichnet, daß man ein Reaktionsgemisch verwendet, das einen partikelförmigen Träger enthält, der mit rekombinanten Antigenen von Borrelia Burgdorferi SSP beschichtet ist.

32. Verfahren nach einem der Ansprüche 1 und 3 bis 14, dadurch gekennzeichnet, daß man ein Reaktionsgemisch verwendet, das einen partikelförmigen Träger enthält, der mit einem rekombinantem Membranantigen des Maedi Visna Virus (MVV) beschichtet ist.

33. Reaktionsgemisch zum Nachweisen eines in einer Probe enthaltenen Antikörpers, insbesondere zur Anwendung beim Durchführen des Verfahrens nach einem der Ansprüche 1 und 3 bis 32, dadurch gekennzeichnet, daß das Reaktionsgemisch in einem flüssigen Medium, insbesondere einer Pufferlösung einen partikelförmigen Träger, an dem ein bezüglich des nachzuweisenden Antikörpers spezifisches Antigen immobilisiert ist, und ein Konjugat aus wenigstens einem zum Fc-Fragment von Antikörpern reaktiven Protein und einem Indikator enthält.

34. Reaktionsgemisch nach Anspruch 33, dadurch gekennzeichnet, daß der Träger aus Latexpartikeln besteht.

35. Reaktionsgemisch nach Anspruch 34, dadurch gekennzeichnet, daß die Latexpartikel aus Polystyrol bestehen.

36. Reaktionsgemisch nach Anspruch 35, dadurch gekennzeichnet, daß die Latexpartikel einen durchschnittlichen Durchmesser von 1µm besitzen.

37. Reaktionsgemisch nach einem der Ansprüche 33 bis 36, dadurch gekennzeichnet, daß der Indikator Gold, Pigment, FITC oder gefärbte Kunststoffteilchen ist.

38. Reaktionsgemisch nach einem der Ansprüche 33 bis 37, dadurch gekennzeichnet, daß das Reaktionsgemisch als Konjugat Protein-A-Gold-Konjugat, Protein-G-Gold-Konjugat, Anti-Antikörper-Gold-Konjugat, Protein-A-Fluorochrom- oder Protein-A+G-Farblatex-Konjugat enthält.

39. Reaktionsgemisch nach einem der Ansprüche 33 bis 38, dadurch gekennzeichnet, daß das Reaktionsgemisch das Konjugat und den partikelförmigen Träger in einer physiologischen Pufferlösung als flüssiges Medium enthält.

40. Reaktionsgemisch nach einem der Ansprüche 33 bis 39, dadurch gekennzeichnet, daß der partikelförmige Träger mit wenigstens einem der nachstehend genannten Antigene beschichtet ist:
natives oder rekombinantes Virus-Antigen,
natives oder rekombinantes Bakterien-Antigen,
natives oder rekombinantes Pilz-Antigen,
natives oder rekombinantes zellspezifisches Antigen.

41. Reaktionsgemisch nach einem der Ansprüche 33 bis 40, dadurch gekennzeichnet, daß der partikelförmige Träger mit wenigstens einem der nachstehend genannten Antigene beschichtet ist:
rekombinantes Membranantigen (gp41-HIV-1),
rekombinantes Hüll-Antigen (p24 HIV-1),
rekombinantes Antigen gp 36 HIV-2 und p 26 HIV-2,
Gemisch aus HIV-1 und HIV-2 rekombinanten Antigenen,
Membranantigen aus Entamoeba histolytica,
Antigenpräparation aus Rinderherpesvirus IBR/IPV,
Antigenpräparation aus dem Felinen Leukämievirus,
Antigenpräparation aus dem Parvo Virus,
Antigenpräparation von Salmonella pulorum,
Antigenpräparation aus Salmonella typhii,
rekombinantes Hepatitis B-Virus Antigen (HBV),
rekombinantes Antigen von Borrelia Burgdorferi SSP,
Antigenpräparation aus dem Epstein Barr Virus (EBV) oder rekombinantes Membranantigen des Maedi Visna Virus (MVV).

42. Reaktionsgemisch nach einem der Ansprüche 33 bis 41, dadurch gekennzeichnet, daß das Reaktionsgemisch als Konjugat Protein-A-Gold-Konjugat und/oder Protein-G-Gold-Konjugat enthält, wobei die Goldpartikel einen mittleren Durchmesser von 20 nm aufweisen.

43. Verfahren zum Herstellen eines Reaktionsgemisches nach einem der Ansprüche 33 bis 42, dadurch gekennzeichnet, daß man Trägerpartikel, insbesondere Latexpartikel, vorzugsweise solche aus Polystyrol mit einem durchschnittlichen Durchmesser von vorzugsweise 1 µm, in einem, vorzugsweise alkalischen, Beschichtungspuffer mit wenigstens einem Antigen beschichtet, daß man die beschichteten Trägerpartikel in einer Pufferlösung, insbesondere einer physiologischen Pufferlösung, in einer Menge von 1% bezogen auf die Pufferlösung suspendiert und daß man der so erhaltenen Suspension wenigstens ein Konjugat aus wenigstens einem bezüglich des Fc-Fragmentes von Antikörpern reaktiven Protein gekoppelt an einen Indikator zumischt.

44. Verfahren nach Anspruch 43, dadurch gekennzeichnet, daß man freie Proteinbindungsstellen der Trägerpartikel mit einem Gemisch aus Trägerproteinen und Detergentien absättigt.

45. Verfahren nach Anspruch 43 oder 44, dadurch gekennzeichnet, daß man je sechzehn Teilen der die beschichteten Trägerpartikel enthaltenden Suspension einen Teil Konjugat zumischt.

46. Verfahren nach einem der Ansprüche 2 bis 14, dadurch gekennzeichnet, daß man das Reaktionsgemisch aus zwei Komponenten bildet, wobei die Komponente I den beschichteten Träger und Konjugat und die Komponente II antigenspezifische monoklonale Antikörper enthält, daß man die Antigenprobe der Komponenten II zugibt, und daß man dem so erhaltenen Gemisch die Komponente I zusetzt.

47. Verfahren nach einem der Ansprüche 2 bis 14 und 46, dadurch gekennzeichnet, daß man bei der Untersuchung einer nicht oder nur geringfügig IgG-Moleküle enthaltenden Antigenprobe, wie beispielsweise Urin eine Komponente II verwendet, die zusätzlich zu dem antigenspezifischen, monoklonalen Antikörper unspezifische IgG-Moleküle, bzw. deren Fc-Fragmente enthält.

48. Verfahren nach Anspruch 46 oder 47, dadurch gekennzeichnet, daß man Komponente I und Komponente II in Form von Lösungen in einem Puffer, insbesondere einem physiologischen Puffer verwendet.

49. Verfahren nach einem der Ansprüche 2 bis 14 und 46 bis 48, dadurch gekennzeichnet, daß man zum Nachweisen von hCG in Urin oder Serum einen partikelförmigen Träger verwendet, der mit Fab- oder F(ab)₂-Fragmenten von einem monoklonalen Anti-hCG-Antikörper beschichtet ist.

50. Verfahren nach einem der Ansprüche 2 bis 14 und 46 bis 48, dadurch ge-kennzeichnet, daß man zum Nachweisen von HIV-1 p24-Antigen einen Träger verwendet, der mit Fab- oder F(ab)₂-Fragmenten von einem monoklonalen Anti-p24-Antikörper beschichtet ist.

51. Verfahren nach einem der Ansprüche 2 bis 14 und 46 bis 48, dadurch gekennzeichnet, daß man zum Nachweisen von Hepatitis-B-Antigen in Serum oder Plasma einen Träger verwendet, der mit Fab- oder F(ab)₂-Fragmenten von einem monoklonalen Anti-Hepatitis-B-Antikörper beschichtet ist.

52. Verfahren nach einem der Ansprüche 2 bis 14 und 46 bis 48, dadurch gekennzeichnet, daß man zum Nachweisen von PMSG einen Träger verwendet, der mit Fab- oder F(ab)₂-Fragmenten von einem monoklonalen Anti-PMSG-Antikörper beschichtet ist.

53. Mittel zum Nachweisen eines in einer Probe enthaltenen Antigens, insbesondere zur Anwendung beim Durchführen des Verfahrens nach einem der Ansprüche 2 bis 14 und 46 bis 52, dadurch gekennzeichnet, daß das Mittel aus einer Komponente I und einer Komponente II besteht, daß die Komponente I einen partikelförmigen Träger, der mit Fab- oder F(ab)₂-Fragmenten eines für das nachzuweisende Antigen spezifischen Antikörpers beschichtet ist, und ein Konjugat enthält, das aus wenigstens einem zum Fc-Fragment von Antikörpern reaktiven Protein und einem Indikator besteht, daß die Komponente II für das zu bestimmende Antigen spezifische, monoklonale Antikörper, die sich in ihrer Epitopspezifität vom immobilisierten Antikörper unterscheiden, und gegebenenfalls bezüglich des nachzuweisenden Antigens unspezifische IgG-Moleküle bzw. deren Fc-Fragmente enthält.

54. Mittel nach Anspruch 53, dadurch gekennzeichnet, daß der Träger aus Latexpartikeln, insbesondere solchen aus Polystyrol besteht.

55. Mittel nach Anspruch 54, dadurch gekennzeichnet, daß die Latexpartikel einen durchschnittlichen Durchmesser von 1 µm besitzen.

56. Mittel nach einem der Ansprüche 53 bis 55, dadurch gekennzeichnet, daß der Indikator Gold, Pigment, FITC oder gefärbte Kunststoffteilchen ist.

57. Mittel nach einem der Ansprüche 53 bis 56, dadurch gekenndaß der Indikator Gold-Partikel mit einem mittleren Durchmesser von 20 nm sind.

58. Mittel nach einem der Ansprüche 53 bis 57, dadurch gekennzeichnet, daß die Komponente I als Konjugat Protein-A-Gold-Konjugat, Protein-G-Gold-Konjugat, Anti-Antikörper-Gold-Konjugat, Protein-A-Fluorochrom-Konjugat oder Protein-A+G-Farblatex-Konjugat enthält.

59. Mittel nach einem der Ansprüche 53 bis 58, dadurch gekennzeichnet, daß die Komponenten I und II Lösungen in einem Puffer, insbesondere einem physiologischen Puffer sind.

60. Mittel nach einem der Ansprüche 53 bis 59, dadurch gekennzeichnet, daß der partikelförmige Träger mit wenigstens einem Fab- oder F(ab)₂-Fragment der nachstehend genannten Antikörper beschichtet ist:
bezüglich viraler Antigene spezifische monoklonale Antikörper,
bezüglich bakterieller Antigene spezifische monoklonale Antikörper,
bezüglich zellulärer und extrazellulärer Antigene spezifische
monoklonale Antikörper,
monoklonale anti-hCG-Antikörper,
monoklonale anti-p24-Antikörper,
anti-Hepatitis-B-Antikörper,
monoklonale anti-PMSG-Antikörper,
bezüglich Mykotoxinen spezifischer monoklonaler Antikörper, bezüglich Tumormarker spezifischer monoklonaler Antikörper, bezüglich menschlicher oder tierischer Hormone spezifische monklonale Antikörper.

## Claims

1. Process for the detection of an antibody contained in a sample by binding the antibody to be detected to an antigen specific to the antibody to be detected, characterised in that the sample is added to and incubated in a reaction mixture containing a particulate carrier, on which an antigen specific to the antibody to be detected is immobilised, and also containing a conjugate composed of at least one protein reactive to the Fc fragment of antibodies and an indicator bound to this, in order to form complexes from conjugate and antibodies and to bind these complexes to the carrier, in that the carrier is then separated, and in that this is examined for the presence of complexes bound to the carrier, said complexes comprising conjugate and the antibody to be detected.

2. Process for the detection of an antigen contained in a sample by binding the antigen to be detected to a Fab or F(ab)₂ fragment of a monoclonal antibody specific to the antigen to be detected, characterised in that the sample is incubated in a reaction mixture containing a particulate carrier, on which Fab or F(ab)₂ fragments of a monoclonal antibody specific to the antigen to be detected are immobilised, also containing a conjugate composed of at least one protein reactive to the Fc fragment of antibodies and an indicator bound to this, as well as containing at least one monoclonal antibody specific to the antigen to be detected, which antibody differs from the immobilised antibody in its epitopic specificity, in order to form aggregates composed of particulate carrier, the antigen to be detected and complexes comprising conjugate and antibodies, and in that this is examined for the presence of aggregates containing the antigen to be detected.

3. Process according to Claim 1 or 2, characterised in that latex particles, in particular those composed of polystyrene, are used.

4. Process according to Claim 3, characterised in that latex particles with an average diameter of 1 µm are used.

5. Process according to one of Claims 1 to 4, characterised in that gold, pigments, FITC, coloured plastic particles are used as indicator.

6. Process according to one of Claims 1 to 5, characterised in that the reaction mixture used contains the conjugate protein A gold conjugate, protein G gold conjugate, anti-antibody gold conjugate, Fc-reactive protein fluorochrome conjugate or protein A+G coloured latex conjugate.

7. Process according to Claim 6, characterised in that a reaction mixture is used which is the conjugate protein A gold conjugate and/or protein G gold conjugate, and in that the gold particles have an average diameter of 20 nm.

8. Process according to one of Claims 1 to 7, characterised in that a reaction mixture is used which contains the reaction partners in a buffer solution, in particular a physiological buffer solution.

9. Process according to one of Claims 1 to 8, characterised in that the carrier is separated by means of filtration.

10. Process according to Claim 9, characterised in that the carrier is separated by means of filtration using a membrane, in particular a cellulose acetate membrane.

11. Process according to Claim 9 or 10, characterised in that filtration is assisted by using a vacuum or by arranging an absorbent cushion under the filter, in particular under the membrane.

12. Process according to one of Claims 1 to 11, characterised in that after the sample has been added to the reaction mixture, incubation is carried out for 2 to 5 min., preferably for 2 min., at a temperature of 19°C to 30°C.

13. Process according to one of Claims 1 to 12, characterised in that the separated carrier is washed, the washing liquid being preferably added to the reaction mixture prior to or during separation of the carrier.

14. Process according to Claim 13, characterised in that washing is carried out by means of a detergent buffer solution.

15. Process according to one of Claims 1 and 3 to 14, characterised in that a carrier is used, on which a native or recombinant viral antigen is immobilised.

16. Process according to one of Claims 1 and 3 to 14, characterised in that a carrier is used, on which a native or recombinant bacterial antigen is immobilised.

17. Process according to one of Claims 1 and 3 to 14, characterised in that a carrier is used, on which a native or recombinant fungal antigen is immobilised.

18. Process according to one of Claims 1 and 3 to 14, characterised in that a carrier is used, on which a native or recombinant cell-specific antigen is immobilised.

19. Process according to one of Claims 1 and 3 to 14, characterised in that a carrier is used, on which a recombinant cell-specific antigen is immobilised.

20. Process according to one of Claims 1 and 3 to 14, characterised in that the reaction mixture used is one containing a particulate carrier, which is coated with recombinant membrane antigen (gp4l HIV-1) and with recombinant sheath antigen (p24 HIV-1).

21. Process according to one of Claims 1 and 3 to 14, characterised in that the reaction mixture used is one containing a particulate carrier, which is coated with gp36 HIV-2 and p26 HIV-2 recombinant antigen.

22. Process according to one of Claims 1 and 3 to 14, characterised in that the reaction mixture used is one containing a particulate carrier, which is coated with a mixture of HIV-1 and HIV-2 recombinant antigens.

23. Process according to one of Claims 1 and 3 to 14, characterised in that the reaction mixture used is one containing a particulate carrier, which is coated with Entamoeba histolytica membrane antigens.

24. Process according to one of Claims 1 and 3 to 14, characterised in that the reaction mixture used is one containing a particulate carrier, which is coated with an antigen preparation composed of IBR/IPV bovine herpes virus, and which contains as the conjugate protein G gold conjugate.

25. Process according to one of Claims 1 and 3 to 14, characterised in that the reaction mixture used is one containing a particulate carrier, which is coated with an antigen preparation composed of the feline leukaemia virus.

26. Process according to one of Claims 1 and 3 to 14, characterised in that the reaction mixture used is one containing a particulate carrier, which is coated with an antigen preparation composed of the swine parvo virus.

27. Process according to one of Claims 1 and 3 to 14, characterised in that the reaction mixture used is one containing a particulate carrier, which is coated with an antigen preparation of Salmonella pulorum.

28. Process according to one of Claims 1 and 3 to 14, characterised in that the reaction mixture used is one containing a particulate carrier, which is coated with an antigen preparation composed of Salmonella typhi.

29. Process according to one of Claims 1 and 3 to 14, characterised in that the reaction mixture used is one containing a particulate carrier, which is coated with recombinant hepatitis B antigen (HBV).

30. Process according to one of Claims 1 and 3 to 14, characterised in that the reaction mixture used is one containing a particulate carrier, which is coated with an antigen preparation composed of Epstein Barr virus (EBV).

31. Process according to one of Claims 1 and 3 to 14, characterised in that the reaction mixture used is one containing a particulate carrier, which is coated with recombinant antigens of Borrelia burgdorferi SSP.

32. Process according to one of Claims 1 and 3 to 14, characterised in that the reaction mixture used is one containing a particulate carrier, which is coated with a recombinant antigen of the Maedi visna virus (MVV).

33. Reaction mixture for detecting the presence of an antibody contained in a sample, in particular to be used for implementation of the process according to one of Claims 1 and 3 to 32, characterised in that the reaction mixture in a liquid medium, in particular a buffer solution, contains a particulate carrier, on which an antigen specific to the antibody to be detected is immobilised, and also contains a conjugate composed of at least one protein reactive to the Fc fragment of antibodies and of an indicator.

34. Reaction mixture according to Claim 33, characterised in that the carrier is composed of latex particles.

35. Reaction mixture according to Claim 34, characterised in that the latex particles are composed of polystyrene.

36. Reaction mixture according to Claim 35, characterised in that the latex particles have an average diameter of 1 µm.

37. Reaction mixture according to one of Claims 33 to 36, characterised in that the indicator is gold, pigment, FITC or coloured plastic particles.

38. Reaction mixture according to one of Claims 33 to 37, characterised in that the reaction mixture used contains the conjugate protein A gold conjugate, protein G gold conjugate, anti-antibody gold conjugate, protein A fluorochrome conjugate or protein A+G coloured latex conjugate.

39. Reaction mixture according to one of Claims 33 to 38, characterised in that the reaction mixture contains the conjugate and the particulate carrier in a physiological buffer solution as liquid medium.

40. Reaction mixture according to one of Claims 33 to 39, characterised in that the particulate carrier is coated with at least one of the following antigens:
- native or recombinant viral antigen,
- native or recombinant bacterial antigen,
- native or recombinant fungal antigen,
- native or recombinant cell-specific antigen.

41. Reaction mixture according to one of Claims 33 to 40, characterised in that the particulate carrier is coated with at least one of the following antigens:
- recombinant membrane antigen (gp4l HIV-1),
- recombinant sheath antigen (p24 HIV-1),
- recombinant gp36 HIV-2 and p26 HIV-2 antigen,
- a mixture of HIV-1 and HIV-2 recombinant antigens,
- membrane antigen composed of Entamoeba histolytica,
- antigen preparation composed of IBR/IPV bovine herpes virus,
- antigen preparation composed of the feline leukaemia virus,
- antigen preparation composed of the parvo virus,
- antigen preparation composed of Salmonella pulorum,
- antigen preparation composed of Salmonella typhi,
- recombinant hepatitis B virus antigen (HBV),
- recombinant antigen of Borrelia burgdorferi SSP,
- antigen preparation composed of Epstein Barr virus (EBV), or
- recombinant membrane antigen of the Maedi visna virus (MVV).

42. Reaction mixture according to one of Claims 33 to 41, characterised in that the reaction mixture contains the conjugate protein A gold conjugate and/or protein G gold conjugate, the gold particles having an average diameter of 20 nm.

43. Process for the production of a reaction mixture according to one of Claims 33 to 42, characterised in that carrier particles, in particular latex particles preferably composed of polystyrene, preferably with an average diameter of 1 µm, are coated with at least one antigen in a preferably alkaline coating buffer, in that the coated carrier particles are suspended in a buffer solution, in particular a physiological buffer solution, to an amount of 1% in relation to the buffer solution, and in that there is added to the suspension obtained thus at least one conjugate composed of at least one protein reactive to the Fc fragment of antibodies attached to an indicator.

44. Process according to Claim 43, characterised in that free protein links of the carrier particle are saturated with a mixture of carrier proteins and detergents.

45. Process according to Claim 43 or 44, characterised in that one part conjugate is added in each case to sixteen parts suspension containing the coated carrier particles.

46. Process according to one of Claims 2 to 14, characterised in that the reaction mixture is formed from two components, component I containing the coated carrier and conjugate and component II containing antigen-specific monoclonal antibodies, in that the antigen sample is added to component II, and in that component I is added to the mixture obtained thus.

47. Process according to one of Claims 2 to 14 and 46, characterised in that during examination of an antigen sample containing none or only a few IgG molecules, such as for example urine, a component II is used, which contains non-specific IgG molecules or their Fc fragments in addition to the antigen-specific monoclonal antibodies.

48. Process according to Claim 46 or 47, characterised in that component I and component II are used in the form of solutions in a buffer, in particular a physiological buffer.

49. Process according to Claims 2 to 14 and 46 to 48, characterised in that to detect hCG in urine or serum, a particulate carrier which is coated with Fab or F(ab)₂ fragments of a monoclonal anti-hCG antibody is used.

50. Process according to one of Claims 2 to 14 and 46 to 48, characterised in that to detect HIV-1 p24 antigen, a carrier coated with Fab or F(ab)₂ fragments of a monoclonal anti-p24 antibody is used.

51. Process according to one of Claims 2 to 14 and 46 to 48, characterised in that to detect hepatitis B antigen in serum or plasma, a carrier coated with Fab or F(ab)₂ fragments of a monoclonal anti-hepatitis B antibody is used.

52. Process according to one of Claims 2 to 14 and 46 to 48 characterised in that to detect PMSG, a carrier coated with Fab or F(ab)₂fragments of a monoclonal anti-PMSG antibody is used.

53. Means for the detection of an antigen contained in a sample, in particular to be used for implementation of the process according to one of Claims 2 to 14 and 46 to 52, characterised in that the means comprises a component I and a component II, in that component I contains a particulate carrier, which is coated with Fab or F(ab)₂ fragments of an antibody specific to the antigen to be detected, and also contains a conjugate composed of at least one protein reactive to the Fc fragment of antibodies and an indicator, in that component II contains monoclonal antibodies specific to the antigen to be detected, which antibodies differ from the immobilised antibodies in their epitopic specificity, and possibly IgG molecules or their Fc fragments which are non-specific to the antigen to be detected.

54. Means according to Claim 53, characterised in that the carrier is composed of latex particles, in particular made from polystyrene.

55. Means according to Claim 54, characterised in that the latex particles have an average diameter of 1 µm.

56. Means according to Claims 53 to 55, characterised in that the indicator is gold, pigment, FITC or coloured plastic particles.

57. Means according to one of Claims 53 to 56, characterised in that the indicator comprises gold particles with an average diameter of 20 nm.

58. Means according to one of Claims 53 to 57, characterised in that component I contains the conjugate protein A gold conjugate, protein G gold conjugate, anti-antibody gold conjugate, protein A fluorochrome conjugate or protein A+G coloured latex conjugate.

59. Means according to one of Claims 53 to 58, characterised in that components I and II are solutions in a buffer, in particular a physiological buffer.

60. Means according to one of Claims 53 to 59, characterised in that the particulate carrier is coated with at least one Fab or F(ab)₂ fragment of the following antibodies:
- monoclonal antibodies specific to viral antigens,
- monoclonal antibodies specific to bacterial antigens,
- monoclonal antibodies specific to cellular and extracellular antigens,
- monoclonal anti-hCG antibodies,
- monoclonal anti-p24 antibodies,
- anti-hepatitis B antibodies,
- monoclonal anti-PMSG antibodies,
- monoclonal antibodies specific to mycotoxins,
- monoclonal antibodies specific to tumour markers,
- monoclonal antibodies specific to human or animal hormones.

## Revendications

1. Procédé pour déceler un anticorps contenu dans un échantillon, par liaison de l'anticorps à déceler à un antigène spécifique, correspondant à l'anticorps à déceler, caractérisé en ce qu'on introduit et incube l'échantillon dans un mélange réactionnel qui contient un support sous forme de particules, sur lequel est immobilisé un antigène spécifique, correspondant à l'anticorps à déceler, et un conjugué constitué d'au moins une protéine apte à réagir avec le fragment Fc des anticorps, et d'un indicateur lié à cette protéine, afin de former des complexes du conjugué et des anticorps et de lier ces complexes au support, en ce que l'on sépare ensuite le support et en ce que l'on recherche la présence de complexes du conjugué et de l'anticorps à déceler, liés au support.

2. Procédé pour déceler un antigène contenu dans un échantillon, par liaison de l'antigène à déceler à un fragment Fab ou F(ab)₂ d'un anticorps monoclonal, spécifique de l'antigène à déceler, caractérisé en ce que l'on incube l'échantillon dans un mélange réactionnel qui contient un support sous forme de particules, sur lequel sont immobilisés des fragments Fab ou F(ab)₂ d'un anticorps monoclonal, spécifique de l'antigène à déceler, et un conjugué qui est constitué d'au moins une protéine apte à réagir avec le fragment Fc des anticorps et d'un indicateur lié à cette protéine, ainsi qu'au moins un anticorps monoclonal spécifique, correspondant à l'antigène à déceler, qui se différencie de l'anticorps immobilisé par sa spécificité de groupement déterminant, afin de former des agrégats constitués du support sous forme de particules, de l'antigène à déceler et des complexes constitués du conjugué et des anticorps, et en ce que l'on recherche la présence d'agrégats qui contiennent l'antigène à déceler.

3. Procédé selon la revendication 1 ou 2, caractérisé en ce que l'on utilise comme support des particules de latex, en particulier celles en polystyrène.

4. Procédé selon la revendication 3, caractérisé en ce que l'on utilise des particules de latex ayant un diamètre moyen de 1 µm.

5. Procédé selon l'une quelconque des revendications 1 à 4, caractérisé en ce que l'on utilise comme indicateur, l'or, des pigments, le FITC, des particules de produit synthétique colorées ou des produits similaires.

6. Procédé selon l'une quelconque des revendications 1 à 5, caractérisé en ce que l'on utilise un mélange réactionnel qui contient comme conjugué, un conjugué protéine A-or, un conjugué protéine G-or, un conjugué anti-anticorps-or, un conjugué protéine apte à réagir avec Fc-fluorochrome, ou un conjugué protéine A+G-latex coloré.

7. Procédé selon la revendication 6, caractérisé en ce que l'on utilise un mélange réactionnel dans lequel le conjugué est un conjugué protéine A-or et/ou un conjugué protéine G-or, et en ce que les particules d'or présentent un diamètre moyen de 20 nm.

8. Procédé selon l'une quelconque des revendications 1 à 7, caractérisé en ce que l'on utilise un mélange réactionnel qui contient les partenaires de la réaction dans une solution tampon, en particulier une solution tampon physiologique.

9. Procédé selon l'une quelconque des revendications 1 à 8, caractérisé en ce que l'on sépare le support par filtration.

10. Procédé selon la revendication 9, caractérisé en ce que l'on sépare le support par filtration, en utilisant une membrane, en particulier une membrane d'acétate de cellulose.

11. Procédé selon la revendication 9 ou 10, caractérisé en ce que l'on facilite la filtration en utilisant le vide ou en disposant un coussin capable d'aspirer sous le filtre, en particulier sous la membrane.

12. Procédé selon l'une quelconque des revendications 1 à 11, caractérisé en ce que, après l'addition de l'échantillon au mélange réactionnel, on incube le tout pendant 2 à 5 minutes, de préférence pendant 2 minutes, à une température de 19 à 30°C.

13. Procédé selon l'une quelconque des revendications 1 à 12, caractérisé en ce qu'on lave le support séparé et ajoute le liquide de lavage au mélange réactionnel, de préférence déjà avant ou pendant la séparation du support.

14. Procédé selon la revendication 13, caractérisé en ce qu'on effectue le lavage à l'aide d'une solution tampon contenant un détergent.

15. Procédé selon l'une quelconque des revendications 1 et 3 à 14, caractérisé en ce qu'on utilise un support sur lequel est immobilisé un antigène de virus, naturel ou recombinant.

16. Procédé selon l'une quelconque des revendications 1 et 3 à 14, caractérisé en ce qu'on utilise un support sur lequel est immobilisé un antigène de bactérie, naturel ou recombinant.

17. Procédé selon l'une quelconque des revendications 1 et 3 à 14, caractérisé en ce qu'on utilise un support sur lequel est immobilisé un antigène de champignon, naturel ou recombinant.

18. Procédé selon l'une quelconque des revendications 1 et 3 à 14, caractérisé en ce qu'on utilise un support sur lequel est immobilisé un antigène spécifique des cellules, naturel ou recombinant.

19. Procédé selon l'une quelconque des revendications 1 et 3 à 14, caractérisé en ce qu'on utilise un support sur lequel est immobilisé un antigène spécifique des cellules, recombinant.

20. Procédé selon l'une quelconque des revendications 1 et 3 à 14, caractérisé en ce qu'on utilise un mélange réactionnel qui contient un support sous forme de particules, qui est recouvert d'antigène de membrane recombinant (gp 41-HIV-1) et d'antigène d'enveloppe recombinant (p 24 HIV-1).

21. Procédé selon l'une quelconque des revendications 1 et 3 à 14, caractérisé en ce qu'on utilise un mélange réactionnel qui contient un support sous forme de particules, qui est recouvert d'antigène recombinant gp 36 HIV-2 et p 26 HIV-2.

22. Procédé selon l'une quelconque des revendications 1 et 3 à 14, caractérisé en ce qu'on utilise un mélange réactionnel qui contient un support sous forme de particules, qui est recouvert d'un mélange d'antigènes recombinants de HIV-1 et HIV-2.

23. Procédé selon l'une quelconque des revendications 1 et 3 à 14, caractérisé en ce qu'on utilise un mélange réactionnel qui contient un support sous forme de particules, qui est recouvert d'antigène de membrane, provenant de Entamoeba histolytica.

24. Procédé selon l'une quelconque des revendications 1 et 3 à 14, caractérisé en ce qu'on utilise un mélange réactionnel qui contient un support sous forme de particules, qui est recouvert d'une préparation d'antigène provenant de virus d'herpès de boeuf IBR/IPV, et qui contient comme conjugué, un conjugué protéine G-or.

25. Procédé selon l'une quelconque des revendications 1 et 3 à 14, caractérisé en ce qu'on utilise un mélange réactionnel qui contient un support sous forme de particules, qui est recouvert d'une préparation d'antigène provenant du virus de la leucémie des félins.

26. Procédé selon l'une quelconque des revendications 1 et 3 à 14, caractérisé en ce qu'on utilise un mélange réactionnel qui contient un support sous forme de particule, qui est recouvert d'une préparation d'antigène provenant du parvovirus du porc.

27. Procédé selon l'une quelconque des revendications 1 et 3 à 14, caractérisé en ce qu'on utilise un mélange réactionnel qui contient un support sous forme de particules, qui est recouvert d'une préparation d'antigène de Salmonella pulorum.

28. Procédé selon l'une quelconque des revendications 1 et 3 à 14, caractérisé en ce qu'on utilise un mélange réactionnel qui contient un support sous forme de particules, qui est recouvert d'une préparation d'antigène provenant de Salmonella typhii.

29. Procédé selon l'une quelconque des revendications 1 et 3 à 14, caractérisé en ce qu'on utilise un mélange réactionnel qui contient un support sous forme de particules, qui est recouvert d'antigène recombinant du virus de l'hépatite B (HBV).

30. Procédé selon l'une quelconque des revendications 1 et 3 à 14, caractérisé en ce qu'on utilise un mélange réactionnel qui contient un support sous forme de particules, qui est recouvert d'une préparation d'antigène provenant du virus d'Epstein Barr (EBV).

31. Procédé selon l'une quelconque des revendications 1 et 3 à 14, caractérisé en ce qu'on utilise un mélange réactionnel qui contient un support sous forme de particules, qui est recouvert d'antigènes recombinants de Borrelia Burgdorferi SSP.

32. Procédé selon l'une quelconque des revendications 1 et 3 à 14, caractérisé en ce qu'on utilise un mélange réactionnel qui contient un support sous forme de particules, qui est recouvert d'un antigène de membrane recombinant du virus Maedi Visna (MVV).

33. Mélange réactionnel pour déceler un anticorps présent dans un échantillon, destiné en particulier à être utilisé lors de la réalisation du procédé selon l'une quelconque des revendications 1 et 3 à 32, caractérisé en ce que ledit mélange réactionnel contient, dans un milieu liquide, en particulier une solution tampon, un support sous forme de particules, sur lequel est immobilisé un antigène spécifique, correspondant à l'anticorps à déceler, et un conjugué constitué d'au moins une protéine apte à réagir avec le fragment Fc des anticorps et d'un indicateur.

34. Mélange réactionnel selon la revendication 33, caractérisé en ce que le support est constitué de particules de latex.

35. Mélange réactionnel selon la revendication 34, caractérisé en ce que les particules de latex sont constituées de polystyrène.

36. Mélange réactionnel selon la revendication 35, caractérisé en ce que les particules de latex présentent un diamètre moyen de 1 µm.

37. Mélange réactionnel selon l'une quelconque des revendications 33 à 36, caractérisé en ce que l'indicateur est l'or, un pigment, le FITC ou des particules de produit synthétique colorées.

38. Mélange réactionnel selon l'une quelconque des revendications 33 à 37, caractérisé en ce que le mélange réactionnel contient comme conjugué, un conjugué protéine A-or, un conjugué protéine G-or, un conjugué anti-anticorps-or, un conjugué protéine A-fluorochrome ou un conjugué protéine A+G-latex coloré.

39. Mélange réactionnel selon l'une quelconque des revendications 33 à 38, caractérisé en ce que le mélange réactionnel contient le conjugué et le support sous forme de particules, dans une solution tampon physiologique en tant que milieu liquide.

40. Mélange réactionnel selon l'une quelconque des revendications 33 à 39, caractérisé en ce que le support sous forme de particules est recouvert d'au moins l'un des antigènes cités ci-après :
un antigène de virus, naturel ou recombinant,
un antigène de bactérie, naturel ou recombinant,
un antigène de champignon, naturel ou recombinant,
un antigène spécifique des cellules, naturel ou recombinant.

41. Mélange réactionnel selon l'une quelconque des revendications 33 à 40, caractérisé en ce que le support sous forme de particules est recouvert d'au moins l'un des antigènes cités ci-après :
l'antigène de membrane recombinant (gp 41-HIV-1),
l'antigène d'enveloppe recombinant (p 24 HIV-1),
l'antigène recombinant gp 36 HIV-2 et p 26 HIV-2,
un mélange d'antigènes recombinants de HIV-1 et HIV-2,
l'antigène de membrane provenant de Entamoeba histolytica,
une préparation d'antigène provenant du virus de l'herpès de boeuf IBR/IPV,
une préparation d'antigène provenant du virus de la leucémie des félins,
une préparation d'antigène provenant du parvovirus,
une préparation d'antigène de Salmonella pulorum,
une préparation d'antigène provenant de Salmonella typhii,
un antigène recombinant du virus de l'hépatite B (HBV),
un antigène recombinant de Borrelia Burgdorferi SSP,
une préparation d'antigène provenant du virus d'Epstein Barr (EBV), et
un antigène de membrane recombinant du virus Maedi Visna (MVV).

42. Mélange réactionnel selon l'une quelconque des revendications 33 à 41, caractérisé en ce que ledit mélange réactionnel contient comme conjugué, un conjugué protéine A-or et/ou un conjugué protéine G-or, les particules d'or présentant un diamètre moyen de 20 nm.

43. Procédé de préparation d'un mélange réactionnel selon l'une quelconque des revendications 33 à 42, caractérisé en ce que l'on recouvre des particules de support, en particulier des particules de latex, de préférence celles en polystyrène ayant un diamètre moyen égal de préférence à 1 µm, d'au moins un antigène, dans un tampon de revêtement, de préférence alcalin, en ce que l'on met les particules de support recouvertes en suspension dans une solution tampon, en particulier une solution tampon physiologique, en une proportion de 1% par rapport à la solution tampon, et en ce que l'on ajoute à la suspension ainsi obtenue, au moins un conjugué constitué d'au moins une protéine apte à réagir avec le fragment Fc des anticorps, associée à un indicateur.

44. Procédé selon la revendication 43, caractérisé en ce qu'on sature les sites de liaison de protéine libres des particules de support, d'un mélange constitué de protéines de support et de détergents.

45. Procédé selon la revendication 43 ou 44, caractérisé en ce qu'on ajoute une partie de conjugué à 16 parties de la suspension contenant les particules de support recouvertes.

46. Procédé selon l'une quelconque des revendications 2 à 14, caractérisé en ce que l'on forme le mélange réactionnel en partant de deux composants, le composant I contenant le support recouvert et le conjugué, et le composant II contenant des anticorps monoclonaux, spécifiques de l'antigène, en ce que l'on ajoute l'échantillon d'antigène au composant II et en ce que l'on ajoute le composant I au mélange ainsi obtenu.

47. Procédé selon l'une quelconque des revendications 2 à 14 et 46, caractérisé en ce que, lors de l'examen d'un échantillon d'antigène ne contenant pas de molécules de IgG ou contenant seulement peu de molécules de IgG, comme par exemple de l'urine, on utilise un composant II qui contient en plus de l'anticorps monoclonal, spécifique de l'antigène, des molécules de IgG non spécifiques ou leurs fragments Fc.

48. Procédé selon la revendication 46 ou 47, caractérisé en ce que l'on utilise les composants I et II sous la forme de solutions dans un tampon, en particulier un tampon physiologique.

49. Procédé selon l'une quelconque des revendications 2 à 14 et 46 à 48, caractérisé en ce que, pour déceler hCG dans de l'urine ou du sérum, on utilise un support sous forme de particules, qui est recouvert de fragments Fab ou F(ab)₂ d'un anticorps monoclonal anti-hCG.

50. Procédé selon l'une quelconque des revendications 2 à 14 et 46 à 48, caractérisé en ce que, pour déceler l'antigène p24 HIV-1, on utilise un support qui est recouvert de fragments Fab ou F(ab)₂ d'un anticorps monoclonal anti-p24.

51. Procédé selon l'une quelconque des revendications 2 à 14 et 46 à 48, caractérisé en ce que, pour déceler l'antigène de l'hépatite B dans du sérum ou du plasma, on utilise un support qui est recouvert de fragments Fab ou F(ab)₂ d'un anticorps monoclonal anti-hépatite B.

52. Procédé selon l'une quelconque des revendications 2 à 14 et 46 à 48, caractérisé en ce que, pour déceler le PMSG, on utilise un support qui est recouvert de fragments Fab ou F(ab)₂ d'un anticorps monoclonal anti-PMSG.

53. Agent pour déceler un antigène présent dans un échantillon, destiné en particulier à être utilisé lors de la réalisation du procédé selon l'une quelconque des revendications 2 à 14 et 46 à 52, caractérisé en ce que ledit agent est constitué d'un composant I et d'un composant II, en ce que le composant I contient un support sous forme de particules, qui est recouvert de fragments Fab ou F(ab)₂ d'un anticorps spécifique de l'antigène à déceler, et un conjugué qui est constitué d'au moins une protéine apte à réagir avec le fragment Fc des anticorps et d'un indicateur, et en ce que le composant II contient des anticorps monoclonaux, spécifiques de l'antigène à déterminer, qui se différencient de l'anticorps immobilisé par leur spécificité de groupement déterminant, et éventuellement des molécules de IgG non spécifiques de l'antigène à déceler, ou leurs fragments Fc.

54. Agent selon la revendication 53, caractérisé en ce que le support est constitué de particules de latex, en particulier de particules de latex de polystyrène.

55. Agent selon la revendication 54, caractérisé en ce que les particules de latex présentent un diamètre moyen de 1 µm.

56. Agent selon l'une quelconque des revendications 53 à 55, caractérisé en ce que l'indicateur est l'or, un pigment, le FITC ou des particules de produit synthétique colorées.

57. Agent selon l'une quelconque des revendications 53 à 56, caractérisé en ce que l'indicateur est constitué de particules d'or ayant un diamètre moyen de 20 nm.

58. Agent selon l'une quelconque des revendications 53 à 57, caractérisé en ce que le composant I contient comme conjugué, un conjugué protéine A-or, un conjugué protéine G-or, un conjugué anti-anticorps-or, un conjugué protéine A-fluorochrome ou un conjugué protéine A+G-latex coloré.

59. Agent selon l'une quelconque des revendications 53 à 58, caractérisé en ce que les composants I et II sont des solutions dans un tampon, en particulier un tampon physiologique.

60. Agent selon l'une quelconque des revendications 53 à 59, caractérisé en ce que le support sous forme de particules est recouvert d'au moins un fragment Fab ou F(ab)₂ des anticorps cités ci-après :
les anticorps monoclonaux spécifiques d'antigènes viraux,
les anticorps monoclonaux spécifiques d'antigènes bactériens,
les anticorps monoclonaux spécifiques d'antigènes cellulaires et extracellulaires,
les anticorps monoclonaux anti-hCG,
les anticorps monoclonaux anti-p24,
les anticorps anti-hépatite-B,
les anticorps monoclonaux anti-PMSG,
les anticorps monoclonaux spécifiques des mycotoxines,
les anticorps monoclonaux spécifiques des marqueurs tumoraux,
les anticorps monoclonaux spécifiques d'hormones humaines ou animales.
